# EUROPEAN PATENT SPECIFICATION

(11) **EP 2 599 541 B1**
(45) Date of publication and mention of the grant of the patent: **03.05.2017**
(21) Application number: 11306604.7
(22) Date of filing: 02.12.2011
(51) Int. Cl.: C07C 29/154, B01J 37/03, B01J 37/06, B01J 37/18, B01J 23/00, B01J 23/80, B01J 35/00, B01J 35/10, B01J 37/00, C01B 3/16, C01F 7/00, C10K 3/04, C01B 33/38, C01G 9/00

(54) **PROCESS FOR PREPARING A WATER GAS SHIFT CATALYST OPERATING AT MEDIUM TEMPERATURES**
VERFAHREN ZUR HERSTELLUNG EINES WASSERGAS-SHIFT-KATALYSATORS FÜR DEN BETRIEB BEI MITTLEREN TEMPERATUREN
PROCÉDÉ DE PRÉPARATION D'UN CATALYSEUR POUR LA RÉACTION DU GAZ À L'EAU FONCTIONNANT À DES TEMPÉRATURES MOYENNES

(43) Date of publication of application: 05.06.2013
(73) Proprietor: L'Air Liquide Société Anonyme pour l'Etude et l'Exploitation des Procédés Georges Claude, 75007 Paris (FR)
(72) Inventor: Brenna, Giuseppe, 85028 Rionero in Vulture (PZ) (IT); Vaccari, Angelo, 40127 Bologna (IT); Basile, Francesco, 88063 Catanzaro (IT); Fornasari, Giuseppe, 40141 Bologna (IT); Faure, Raphael, 91140 Villebon sur Yvette (FR); Gary, Daniel, 78180 Montigny le Bretonneux (FR)
(74) Representative: Beroud, Amandine

(56) References cited:
- NL-A- 7 115 455
- US-A- 4 436 833
- US-A- 4 835 132
- US-A1- 2005 002 858
- US-A1- 2007 034 552
- US-A1- 2010 112 397
- VELU S ET AL: "SELECTIVE PRODUCTION OF HYDROGEN BY PARTIAL OXIDATION OF METHANOL OVER CATALYSTS DERIVED FROM CUZNAL-LAYERED DOUBLE HYDROXIDES", CATALYSIS LETTERS, SPRINGER NEW YORK LLC, UNITED STATES, vol. 62, no. 2-04, 1 January 1999 (1999-01-01), pages 159-167, XP001027077, ISSN: 1011-372X, DOI: 10.1023/A:1019023811688

## Description

The invention relates to synthesis gas productions and more particularly to a process for preparing a water gas shift catalyst which is able to be operated at medium temperatures.

The water gas shift (WGS) reaction plays a key role in the steam reforming (SR) plants, increasing the hydrogen production and reducing the carbon monoxide content in the exit stream [M.V. Twigg (Ed.), Catalyst Handbook, 2nd ed., Wolfe, London (UK), 1989].
This reaction is currently performed in two steps: a higher temperature step (> 350 °C) at the exit of the steam reformer involving iron-based catalysts stabilized by chromium dioxide, and a low temperature step (at about 250°C) involving copper-based catalysts [K. Klier, "Advances in Catalysis" (D.D. Eley, H. Pines and P.B. Weisz, Eds), Vol. 31, Academic Press, New York, 1982, p. 243; P. Courty and C. Marcilly, "Preparation of Catalysts III" (G. Poncelet, P. Grange and P.A. Jacobs, Eds), Elsevier, Amsterdam (NL), 1983, p. 485]. In the former step, the main objective is to achieve a very low selectivity in the methanation reaction, which is thermodynamically favoured, since the low catalyst activity is compensated both by the temperature and by high carbon monoxide (CO) content. In the latter case, the key objective is to finalize the carbon monoxide conversion, the methanation reaction being significantly reduced at the low temperature.

Hydrotalcite-type (HT) anionic clays have been widely employed as catalysts or catalyst support, on account of the specific and interesting properties of the phases which are obtained by the calcination of the said HT anionic clays, such as a high surface area and homogeneity, a high thermal stability also after reduction, etc.). In particular, Copper-based catalysts obtained from Hydrotalcite-type precursors have been widely used in the synthesis of methanol or of higher molecular weight (HMW) alcohols [P. Courty and C. Marcilly, "Preparation of Catalysts III" (G. Poncelet, P. Grange and P.A. Jacobs, Eds), Elsevier, Amsterdam (NL), 1983, p. 485; F. Cavani et al., Catal. Today 11 (1991), 173; F. Basile and A. Vaccari, "Layered Double Hydroxides - Present and Future" (V. Rives, Ed.), Nova Science, New York (USA), 2001, p. 285; S. Velu et al., Catal. Letters 62 (1999), 159; S. Velu et al, Chem. Commun. (1999), 2341; S. Velu et al. Appl. Catal. A213 (2001), 47; G. Fornasari et al., "Catalysis and Automotive Pollution Control" (A. Cruq and A. Frennet, Eds.), Elsevier, Amsterdam (NL), 1987, 469; A.-M. Hilmen, Appl. Catal. 169 (1998), 355; K.J. Smith and R.B. Anderson, Can. J. Chem. Eng. 61 (1983) 40].

The use of Cu-containing catalysts obtained from Hydrotalcite-type precursors in the WGS reaction performed at low temperature has been also widely reported in the patent literature [US 4,835,132; WO 2003/082,468 A1; US 2010/ 0,102,278 A1, US 2010/0,112,397 A1], evidencing some common features, such as high amounts of copper, to improve the activity, and high amount of aluminium, frequently present and/or added as side phases, to improve the stability with time on stream. For this latter purpose also lanthanum, cerium or zirconium were added. The use of a combination of zinc aluminate and zinc oxide, obtained by calcination of a Zn/Al HT precursor, after doping with significant amounts of alkali (Na, K or Cs), in the water gas shift reaction, is disclosed in EP 2,141,118 A1.
Document US 4 835 132 discloses a synthesis process of a hydrotalcite-type compound comprising the steps (A) mixing 100 g of cupric nitrate trihydrate, 150g zinc nitrate hexahydrate and 110 g aluminium nitrate nonahydrate in an aqueous solution, (B) mixing said solution with an aqueous solution of sodium carbonate, the pH being maintained at 9, to produce precipitate, (C) isolating the precipitate by filtration, washing and drying the precipitate to form a hydrotalcite-type compound.
Document US 2010/112397 discloses a synthesis process of a hydrotalcite-type compound comprising the steps (A) mixing 4,8 g of copper nitrate trihydrate, 6,9 g zinc nitrate hexahydrate and 8,6 g aluminium nitrate nonahydrate in an aqueous solution, (b) mixing said solution with an aqueous solution of sodium carbonate, the pH being maintained at 7,5-8, to produce precipitate, (C) isolating the precipitate by filtration, washing and drying the precipitate to form a hydrotalcite-type compound.
Document Velu S et AL: "Selective production of hydrogen by partial oxidation of methanol over catalysts derived from cuznal-layered double hydroxides", Catalysis Letters, Springer New York LLC, United States, vol. 62, no. 2-04, 1 January 1999 (1999-01-01), pages 159-167, XP001027077, ISSN: 1011-372X, DOI: 10.1023/A:1019023811688 discloses a synthesis process of a hydrotalcite-type compound comprising the steps (A) mixing copper nitrate, 6,9 g zinc nitrate and 8,6 g aluminium nitrate in an aqueous solution, (B) mixing said solution with an aqueous solution of sodium carbonate and sodium hydroxide, the pH being maintained at about 9, to produce a precipitate, (C) isolating the precipitate by filtration, washing and drying the precipitate to form a hydrotalcite-type compound.
Document US 4 436 833 discloses a hydrotalcite-type compound with the general composition (CuₓZn_{y})Al₂(OH)₁₆CO₃*H₂O, wherein x and y have values of from 0,5 to 5,5 and the sum of X and y is 6.
Document NL 7 115 455 discloses an hydrotalcite-type compound with the general composition CuZn₅Al₂(OH)₁₆CO₃*4H₂O.

The inventors thus developed an original catalyst, which exhibits a superior activity in terms of carbon monoxide conversion, hydrogen formation H₂ yield and carbon dioxide selectivity in medium temperature shift (MTS) operating conditions, without forming of by-products. Moreover said catalyst also exhibits a very good stability upon time-on-stream.

The present invention concerns the preparation of a catalyst active in medium temperature shift conditions having a formulation based on an Hydrotalcite-type (HT) precursor containing copper and either carbonate, or silicate as interlayer anions and having a specific phase distribution after calcination and a good Cu dispersion without sintering phenomenon by appropriate temperature reduction treatment. The catalyst is obtained by calcination and reduction of Cu/Zn/Al Hydrotalcite-type precursors synthesized by co-precipitation method, with high Cu dispersion.

The catalysts are active and selective in medium temperature shift (MTS) reaction (at about 300°C), with selectivity in CO₂ and H₂ close to 100%, i.e. without by-products such as methanol or other oxygenate compounds. The catalysts are active and stable between 250°C and 350°C and operating at low residence times, reaching the equilibrium values already at 300°C with a residence time of only 1.0 sec.

The invention also relates to the possibility to obtain high activity, selectivity and stability of Cu-containing catalysts obtained by reduction of specific mixed oxides in which the Cu²⁺ ion is present after calcination of the precursor. Layered hydrotalcite-type anionic clays have been used as precursors to obtain new catalysts, with unusual properties due to the presence of all active elements well dispersed inside brucite-like layers of the precursors. Hydrotalcite-type phases form, by controlled calcination, mixed oxides with high thermal stability, surface area and active-phase dispersion, factors directly affecting the catalytic activity. Hydrotalcite-type precursors are prepared by co-precipitation of all the elements to obtain homogeneous precipitates.

The study refers to stable formulations that not only may exhibit good physicochemical properties in the medium temperature shift conditions, but also may act as active phase in the water gas shift reaction. A first embodiment not part of the invention relates to an hydrotalcite-type compound of the formula (I):

[CuₓZn_{y}Al_{w}(OH)₂] ^{(2x+2y+3w-2)+} (A²⁻)_{(2x+2y+3w-2)/2,} k H₂O (I)

Wherein:
- (A²⁻) represents either a carbonate anion or a silicate anion,
- x > 0,
- y > 0,
- w > 0,
- (x + y) = (1-w),
- 1 < [(x+y)/w] < 5, and
- 1/99 ≤ x/y ≤ 1/1.

According to a particular embodiment, not part of the invention, the atomic ratio x/y ≤ 1/2, in the formula (I) as hereinbefore defined.

According to a particular embodiment, not part of the invention, the atomic ratio x/y ≤ 1/5, in the formula (I) as hereinbefore defined.

According to another particular embodiment, not part of the invention, the atomic ratio x/y ≥1/10, in the formula (I), as hereinbefore defined.

According to another particular embodiment, not part of the invention, the atomic ratio [(x+y)/w] is higher or equal to 2, in the formula (I), as hereinbefore defined.

According to another particular embodiment, not part of the invention, the atomic ratio [(x+y)/w] is less or equal to 3, in the formula (I), as hereinbefore defined.

According to a more particular embodiment, not part of the invention, the hydrotalcite-type compound of the formula (I), as hereinbefore defined is of one of the following formulas:

[Cu_{0.075}Zn_{0.675}Al_{0.25} (OH)₂]^{0.25}+(CO₃²⁻)_{0.125} k H₂O,

[Cu_{0.150}Zn_{0.600}Al_{0.25} (OH)₂]^{0.25+}(CO₃²⁻)_{0.125} k H₂O,

[Cu_{0.066}Zn_{0.600}Al_{0.333} (OH)₂]^{0.25+}(CO₃²⁻)_{0.125} k H₂O,

[Cu_{0.134}Zn_{0.532}Al_{0.333} (OH)₂]^{0.31+}(CO₃²⁻)_{0.155} k H₂O.

According to another embodiment, not part of the invention, a synthesis process of the Hydrotalcite-type compound of the formula (I) as hereinbefore defined, comprises the following steps:
- A step A during which an aqueous solution containing together, aluminium nitrate, zinc nitrate and copper nitrate is prepared by mixing said nitrate salts in the desired molar proportions, with water;
- A step B during which, said solution obtained at step A is mixed with an aqueous solution of sodium carbonate, the pH being maintained between 8 and 10, preferably around 9, to produce a precipitate;
- A step C during which, said precipitate obtained at step B, is isolated by filtration, washed and then dried to form the expected hydrotalcite-type compound of said formula (I).

According to a particular embodiment, not part of the invention, the synthesis process as hereinabove defined further comprises:
- A step D during which said hydrotalcite-type compound of said formula (I) as hereinabove defined, obtained at step C, is grinded to form a powder of particles of said hydrotalcite-type compound of said formula (I).

According to another embodiment, not part of the invention, a compound is characterized in that it is obtained by a process comprising the following steps:
- A step F during which the powder of the hydrotalcite-type compound of the formula (I) as hereinbefore defined is calcined;
- A step G during which the calcined powder obtained at step F is reduced with hydrogen at a temperature of less than 230°C.

According to another embodiment, not part of the invention, the use of the compound obtained by the process as hereinabove defined, is as a catalyst for the water gas shift reaction of synthesis gas. The invention is defined by the appended claims.

The following paragraphs disclose non-limitative examples of catalysts.

### A] Synthesis of catalysts

### Example 1: preparation of catalyst ZAC1c

This example illustrates the preparation process of 20 grams of catalyst containing 10 wt.% of copper and having an atomic ratio (Cu+Zn)/Al = 3, using a carbonate as an intercalated anion in the hydrotalcite-type precursor.

A copper, zinc and aluminium salts dimolar (2M) aqueous solution is prepared from 7.321g of 99.99% copper nitrate hemipentahydrate [Cu(NO₃)₂, 2.5H₂O], 51.165g of 98.00% zinc nitrate hexahydrate [Zn(NO₃)₂, 6H₂O] and 26.016g of 98.00% aluminium nitrate nonahydrate [Al(NO₃)₃, 9H₂O] in about 136.0cm³ of deionised water.

This solution is then drop by drop poured, under energetic magnetic stirring, into a molar (1 M) solution containing 14.479g of 99.50% of sodium carbonate (Na₂CO₃) in about 136.0cm³ of deionised water at 60°C, while maintaining the pH to 9.0±0.1, by a drop by drop addition of a trimolar (3M) aqueous solution of sodium hydroxide (NaOH).

The obtained precipitate is aged well dispersed in the same conditions (at 60°C and pH = 9.0) under energetic magnetic stirring for 45min. The solid precipitate is then separated from the mother liquor by filtration with a Buchner funnel, vacuum being provided by a Venturi water suction device.

The resulting solid corresponds to a hydrotalcite-type compound of the formula:

[Cu_{0.117}Zn_{0.629}Al_{0.254} (OH)₂]^{0.25+}(CO₃²⁻)_{0.125} k H₂O,

This resulting solid is then washed with abundant hot water (60°C, 400 cm³/g of sample) and dried overnight at 70°C. After grinding the obtained precursor, the powder is calcined at 550°C (10°C/min) in a muffle furnace for 6h and then formed and sieved with a size from 30 to 40mesh.

### Example 2: preparation of catalyst ZAC2c

The preparation process is worked as in Example 1 except for the Cu content (wt.%), which is two times more than in Example 1 (ZAC1c).

The metal salts 2M aqueous solution is prepared from 14.641g of 99.99% [Cu(NO₃)₂, 2.5H₂O], 42.000g of 98.00% [Zn(NO₃)₂, 6H₂O] and 26.092g of 98.00% [Al(NO₃)₃, 9H₂O] in about 136.0cm³ of deionised water.

This solution is then drop by drop poured, under energetic magnetic stirring, into a 1 M solution containing 14.521 g of 99.50% of Na₂CO₃ in about 136.0cm³ of deionised water at 60°C, while maintaining the pH to 9.0±0.1, by a drop by drop addition of a 3M aqueous solution of NaOH.

The obtained precipitate is aged well dispersed in the same conditions (at 60°C and pH = 9.0) under energetic magnetic stirring for 45min. The solid precipitate is then separated from the mother liquor by filtration with a Buchner funnel, vacuum being provided by a Venturi water suction device.

The resulting solid corresponds to a hydrotalcite-type compound of the formula:

[Cu_{0.234}Zn_{0.514}Al_{0.252}(OH)₂]^{0.25+}(CO₃²⁻)_{0.125} k H₂O,

This resulting solid is washed with abundant hot water (60°C, 400 cm³/g of sample) and dried overnight at 70°C. After grinding the obtained precursor, the powder is calcined at 550°C (10°C/min) in a muffle furnace for 6h and then formed and sieved with a size from 30 to 40mesh.

### Example 3: preparation of catalyst ZAC3c

The preparation process is worked as in Example 1 except for the Cu content (wt.%), which is three times more than in Example 1 (ZAC1c).

The metal salts 2M aqueous solution is prepared from 21.962g of 99.99% [Cu(NO₃)₂, 2.5H₂O], 32.837g of 98.00% [Zn(NO₃)₂, 6H₂O] and 26.167g of 98.00% [Al(NO₃)₃, 9H₂O] in about 137.0cm³ of deionised water.

This solution is then drop by drop poured, under energetic magnetic stirring, into a 1 M solution containing 14.563g of 99.50% of Na₂CO₃ in about 136.0cm³ of deionised water at 60°C, while maintaining the pH to 9.0±0.1, by a drop by drop addition of a 3M aqueous solution of NaOH.

The obtained precipitate is aged well dispersed in the same conditions (at 60°C and pH = 9.0) under energetic magnetic stirring for 45min. The solid precipitate is then separated from the mother liquor by filtration with a Buchner funnel, vacuum being provided by a Venturi water suction device.

The resulting solid correspond to a hydrotalcite-type compound of the formula:

[Cu_{0.349}Zn_{0.399}Al_{0.251} (OH)₂]^{0.25+}(CO₃²⁻)_{0.125} k H₂O,

The resulting solid is washed with abundant hot water (60°C, 400 cm³/g of sample) and dried overnight at 70°C. After grinding the obtained precursor, the powder is calcined at 550°C (10°C/min) in a muffle furnace for 6h and then formed and sieved with a size from 30 to 40mesh.

### Example 4: preparation of catalyst ZAC1s

The preparation process is worked as in Example 1, except that the Na₂CO₃ solution being replaced by a sodium silicate (Na₂SiO₃) solution.

The metal salts 2M aqueous solution is prepared from 7.321g of 99.99 % [2Cu(NO₃)₂, 5H₂O], 51.165g of 98.00% [Zn(NO₃)₂, 6H₂O] and 26.016g of 98.00% [Al(NO₃)₃, 9H₂O] in about 136.0cm³ of deionised water.

This solution is then drop by drop poured, under energetic magnetic stirring, into a 1 M aqueous solution of sodium silicate (Na₂SiO₃) prepared from 9.103g of sodium tri-silicate solution (27.0% SiO₂) in 41.0cm³ of deionised water, at 60°C, while maintaining the pH to 9.0±0.1, by a drop by drop addition of a 3M aqueous solution of NaOH.

The obtained precipitate is aged well dispersed in the same conditions (at 60°C and pH = 9.0) under energetic magnetic stirring for 45min. The solid precipitate is then separated from the mother liquor by filtration with a Buchner funnel, vacuum being provided by a Venturi water suction device.

The resulting solid correspond to a hydrotalcite-type compound of the formula:

[Cu_{0.117}Zn_{0.629}Al_{0.254} (OH)₂]^{0.25+}(SiO₃²⁻)_{0.125} k H₂O,

This resulting solid is washed with abundant hot water (60°C, 400 cm³/g of sample) and dried overnight at 70°C. After grinding the obtained precursor, the powder is calcined at 550°C (10°C/min) in a muffle furnace for 6h and then formed and sieved with a size from 30 to 40mesh.

### Example 5: preparation of catalyst ZAC2s

The process of Example 2 is followed except that the carbonate solution is replaced by the silicate one.

The metal salts (2M) aqueous solution is prepared from 21.962g of 99.99% [2Cu(NO₃)₂, 5H₂O], 32.837g of 98.00% [Zn(NO₃)₂, 6H₂O] and 26.167g of 98.00% [Al(NO₃)₃, 9H₂O] in about 137.0cm³ of deionised water.

This solution is then drop by drop poured, under energetic magnetic stirring, into a 1M aqueous solution of Na₂SiO₃ prepared from 9.077g of sodium tri-silicate solution (27.0% SiO₂) in 41.0cm³ of deionised water, at 60°C, while maintaining the pH to 9.0±0.1, by a drop by drop addition of a 3M aqueous solution of NaOH.

The obtained precipitate is aged well dispersed in the same conditions (at 60°C and pH = 9.0) under energetic magnetic stirring for 45min. The solid precipitate is then separated from the mother liquor by filtration with a Buchner funnel, vacuum being provided by a Venturi water suction device.

The resulting solid corresponds to a hydrotalcite-type compound of the formula:

[Cu_{0.234}Zn_{0.514}Al_{0.252} (OH)₂]^{0.25+}(SiO₃²⁻)_{0.125} k H₂O,

This resulting solid is washed with abundant hot water (60°C, 400 cm³/g of sample) and dried overnight at 70°C. After grinding the obtained precursor, the powder is calcined at 550°C (10°C/min) in a muffle furnace for 6h and then formed and sieved with a size from 30 to 40mesh.

### Example 6: preparation of catalyst ZAC1cK

The preparation process is worked as in Example 1; however, after the calcination a potassium carbonate (2 wt.% K) (K₂CO₃) solution is used to dope the catalyst by Incipient Wetness Impregnation (IWI) method.

A copper, zinc and aluminium salts (2M) aqueous solution is prepared from 7.321g of 99.99% [2Cu(NO₃)₂, 5H₂O], 51.165g of 98.00% [Zn(NO₃)₂, 6H₂O] and 26.016g of 98.00% [Al(NO₃)₃, 9H₂O] in about 136.0cm³ of deionised water.

This solution is then drop by drop poured, under energetic magnetic stirring, into a 1 M aqueous solution containing 14.479g of 99.50% Na₂CO₃ in about 136.0cm³ of deionised water at 60°C, while maintaining the pH to 9.0±0.1, by a drop by drop addition of a 3M aqueous solution of NaOH.

The obtained precipitate is aged well dispersed in the same conditions (at 60°C and pH = 9.0) under energetic magnetic stirring for 45min. The solid precipitate is then separated from the mother liquor by filtration with a Buchner funnel, vacuum being provided by a Venturi water suction device.

The resulting solid is washed with abundant hot water (60°C, 400 cm³/g of sample) and dried overnight at 70°C. After grinding the obtained precursor, the powder is calcined at 550°C (10°C/min) in a muffle furnace for 6h and then formed and sieved with a size from 30 to 40mesh.

A potassium solution is prepared from 0.31 g of K₂CO₃ in 25cm³ of deionised
water.

The calcined sample is then impregnated with this potassium solution by the IWI technique, dried again at 120 °C for 2 h and, then calcined at 550 °C (10 °/min) in a muffle furnace for 2 h.

### Comparative example: Commercial MTS catalyst

The comparative example is a commercial Cu-based catalyst, optimized to perform the water gas shift reaction at low and/or at medium temperature.

### B] Catalysts characterization

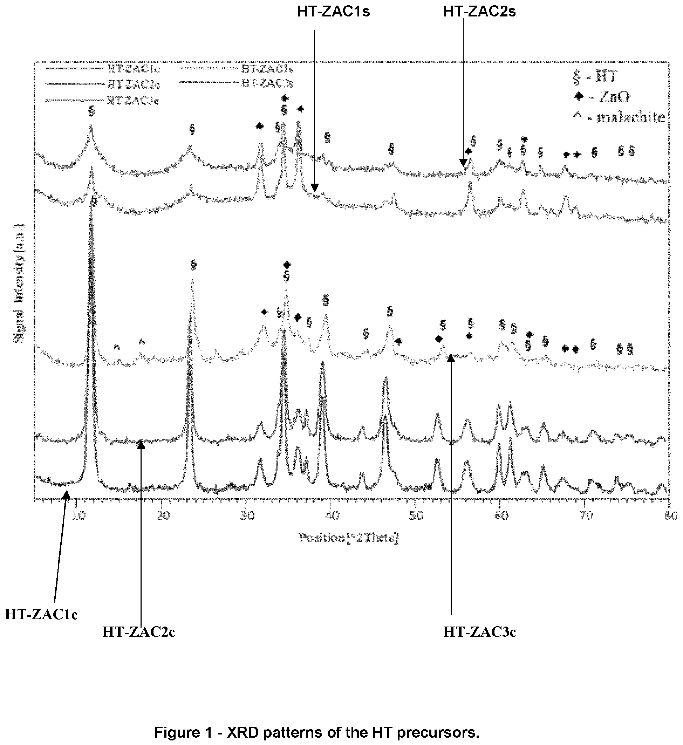

**Figure 1** shows the graphs resulting from the XRD analysis of each of the hydrotalcite-type precursors of catalysts ZAC1c, ZAC2c, ZAC3c, ZAC1s and ZAC2s (identified as HT-ZAC1c, HT-ZAC2c, HT-ZAC3c, HT-ZAC1s and HT-ZAC2s).

The presence of carbonate or silicate ions, in particular the first one, during precipitation results in the HT-structure formation, as identified by XRD analysis. The curve of HT-ZAC3c also has small reflections which can be attributed to a malachite-like phase.

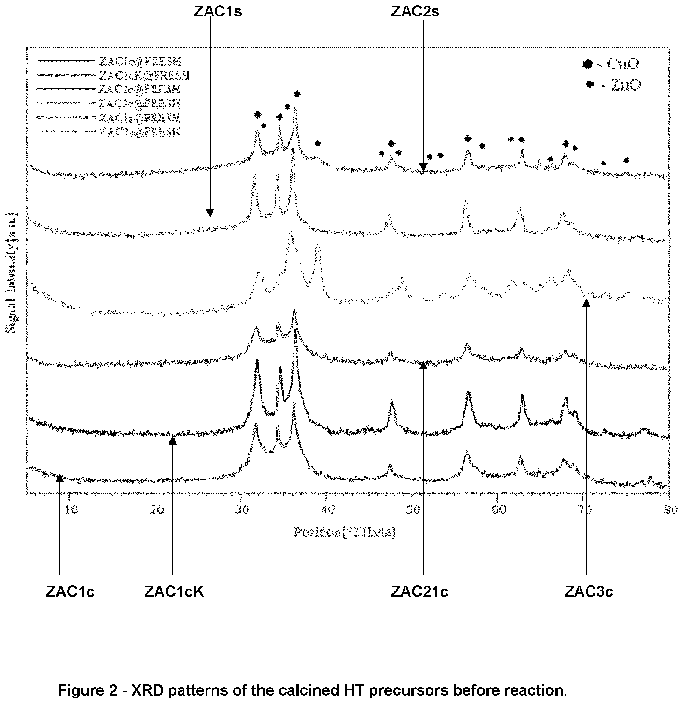

As shown on the graphs of **Figure 2**, the HT-structure containing carbonate ions topotactically evolves after calcination, and XRD shows only a ZnO-like phase without any segregation of Cu-containing species, except for the sample with the highest amount of copper (ZAC3c), which shows also the CuO phase. On the contrary ZnO-like phase is the main phase when silicate ion is used as intercalated anion in the HT-precursor preparation, both before and after calcination.

A summary of the chemical-physical properties is reported in Tables 1 a and 1 b.

The ZAC1cK sample does not show any differences, before and after reaction, in terms of both XRD phases and BET surface values. In the other cases, the effect of sintering is confirmed by the surface area values, which decrease after reaction almost for all samples and is more evident in ZAC1c and ZAC1s samples (the lowest Cu content).

**Table 1a**

| Sample | | Phases | S_{BET}[m²/g] | MSA^{b} [m²/g_{CAT}] | S_{Cu}^{b} [m²/g_{Cu}] |
|---|---|---|---|---|---|
| ZAC1c | Fresh | ZnO | 62 | 3.5 | 35 |
| | Used | Cu⁰ (tr), ZnO, spinel | 34 | 3.1 | 31 |
| ZAC1cK | Fresh | ZnO | 50 | 2.7 | 27 |
| | Used | CuO, ZnO, spinel | 34 | 2.1 | 21 |
| ZAC2c | Fresh | ZnO | 48 | 4.9 | 25 |
| | Used | Cu⁰, ZnO, spinel | 34 | 5.1 | 25 |
| ZAC3c | Fresh | CuO, ZnO | 22 | 3.8 | 13 |
| | used | Cu⁰, CuO, ZnO, spinel (tr) | 18 | 3.9 | 13 |
| ZAC1s | Fresh | CuO, ZnO | 129 | 4.7 | 47 |
| | Used | CuO, ZnO, spinel (tr) | 63 | 3.7 | 37 |
| ZAC2s | Fresh | CuO, ZnO | 110 | 7.1 | 35 |
| | Used | CuO, ZnO, spinel (tr) | 110 | 6.8 | 34 |

**Table 1b**

| Sample | | Phases | d_{Cu}*[nm] | d_{ZnO}^{a} [nm] | D^{b} [%] |
|---|---|---|---|---|---|
| ZAC1c | Fresh | ZnO | 19^{b} | 15 | 5.4 |
| | Used | Cu⁰ (tr), ZnO, spinel | 21^{b} | 20 | 4.8 |
| ZAC1cK | Fresh | ZnO | 25^{b} | 21 | 4.1 |
| | Used | CuO, ZnO, spinel | 32^{b} | 35 | 3.3 |
| ZAC2c | Fresh | ZnO | 27^{b} | 15 | 3.8 |
| | Used | Cu⁰, ZnO, spinel | 12^{a} (Cu), 27^{b} | 16 | 3.5 |
| ZAC3c | Fresh | CuO, ZnO | 20^{a} (CuO), 51^{b} | 20 | 2.0 |
| | used | Cu⁰, CuO, ZnO, spinel (tr) | 14^{b} | 32 | 2.0 |
| ZAC1s | Fresh | CuO, ZnO | 18^{b} | 28 | 7.2 |
| | Used | CuO, ZnO, spinel (tr) | 63 | 20 | 5.7 |
| ZAC2s | Fresh | CuO, ZnO | 19^{b} | 21 | 5.4 |
| | Used | CuO, ZnO, spinel (tr) | 16^{a} (CuO), 20^{b} | 6.8 | 5.3 |

| | | | | | |
|---|---|---|---|---|---|
| a = measured by Debey-Scherrer formula; b = measured by N₂O chemisorption; * = CuO (before reaction); CuO and Cu⁰ (after reaction) | | | | | |

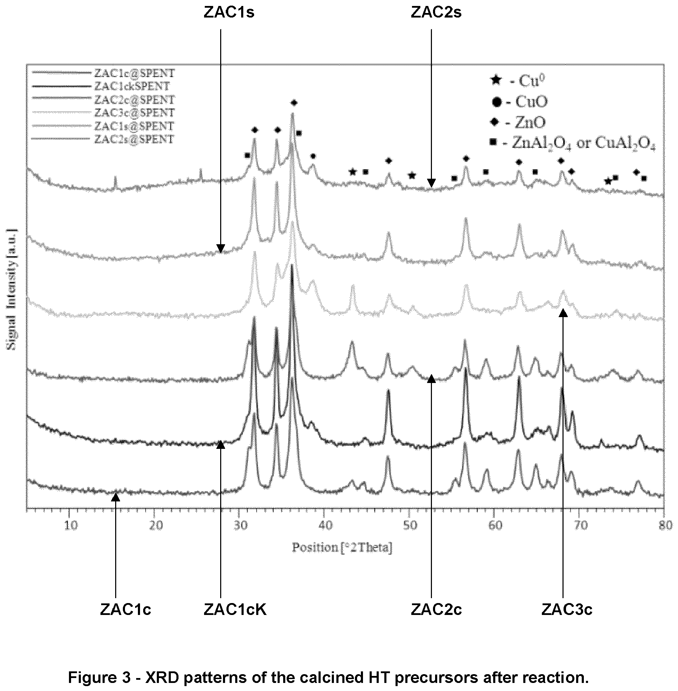

As shown on the graphs of **Figure 3**, the characteristic reflections of Cu⁰ at 43.3, 50.5 and 74.1° are observed in the Cu-containing samples, confirming the catalyst activation. It happens mainly by increasing the content of Cu or after reaction.

The crystal size of the Cu-containing samples, before and after reaction, are calculated by the Debey-Scherrer formula [H. P. Klug and L.E. Alexander, X-Ray Diffraction Procedures, Wiley, New York (USA), 1974], by using the best resolved reflections: at 36.2° for ZnO, 38.9° for CuO and at 43.3° for Cu⁰.

The samples show an increase of crystal size after reaction, evidencing a slight structural sintering of copper and support. A sintering effect is evident in the Cu-containing samples after reaction, by increasing the Cu content.

The pore size distributions (3-40 nm) and the isotherms, grouped into TYPE IV (hysteresis loop of TYPE H4) of the IUPAC classification [IUPAC. Pure Appl. Chem. 57 (4) (1985), 603], indicate that the calcined HT catalysts contain mainly mesopores.

The HT precursors after calcination have been reduced before the catalytic tests under MTS conditions, to obtain the main active phase. During the reduction step, the hot spot temperature is controlled at 220 °C and never should be allowed to exceed 230°C. The N₂ pressure is 10⁶Pa (10bar) with a gas hourly space velocity (GHSV) between 300h⁻¹ and 400h⁻¹.

A typical procedure comprises:
- 1) To remove oxygen (O₂) by purging nitrogen (N₂) in the reactor and, after that, to heat the catalyst to 175°C (50°C/h);
- 2) To add a flow of 0.8 v/v% of hydrogen (H₂), when the temperature of 175°C is reached and kept constant;
- 3) To increase step by step the H₂ concentration with step of 0.2 v/v% without exceeding 1.2 v/v% of H₂/N₂ at this stage for 18 h;
- 4) To increase the inlet temperature up to 220°C (15 °C/h), avoiding to exceed 1.5 v/v% of H₂ in N₂;
- 5) To increase step by step the H₂ concentration up to 4 v/v% in N₂, with step of 0.5 v/v% and, when the temperature of 220°C is reached and stabilized, to check that the catalyst hot spot temperature does not exceed 230 °C. If the catalyst hot spot exceeds 250°C, the protocol must be interrupted (injection of H₂/N₂ stopped, depressurization of the reactor and sweeping by fresh N₂).
- 6) The reduction is considered complete when a H₂ consumption of less than 0.2 v/v% is consecutively assessed during 2h.

For the Cu-containing samples, it is possible to hypothesize the presence of different Cu-containing species on the basis of the literature data [J.D. Stroupe, J. Am. Chem. Soc. 71 (1949), 569], with different oxide-oxide surface interactions. The H₂-TPR profiles of all calcined ZAC catalysts show that, before and after reaction, they are completely reduced at temperatures below 300 °C with a peak typical of the reduction of the highly dispersed Cu species [W.R.A.M. Robinson and J.C. Mol, Appl. Catal. 60 (1990), 61; G.L. Castiglioni, Appl. Catal. 123 (1995), 123; J. Als-Nielsen et al., Nucl. Instrum. Methods Phys. Res. B97 (1995), 522; T.L. Reitz et al., J. Catal. 199 (2001), 193].

ZnO-like phase does not reduce under the experimental conditions, in agreement to the literature [Y.Okamoto et al., J. Phys. Chem. 87 (1983), 3740.], such as confirmed by the ZA3K sample that did not reduce under the analysis conditions.

### C] Catalytic activity test

The catalysts together with the reference catalyst (ZA3K) are shaped as pellets with size between 30 and 40mesh and tested in a plug-flow reactor.

The tubular reactor is heated by an oven in order to have a temperature between 250°C and 350°C (±1°C), measured immediately at the exit of the catalytic bed, and pressurized under 15x10⁵ Pa (15bar).

Dry Gas (DG) and Steam (S) are pre-heated at around 215°C and mixed (mass flow controller) before passing over the catalyst. In order to determine the activity of the catalysts produced by the various examples, in medium temperature shift (MTS) operating conditions, using a typical DG composition containing 18.8 vol% carbon monoxide, 4.6 vol% carbon dioxide, 4.6 vol% methane and hydrogen up to 100vol%. This gas flow and steam are passed over the pre-reduced catalysts with a steam to dry gas v/v ratio (S/DG) of 0.55 and 0.25. Concentration of all components is regularly measured both inlet and exit dry gas by means of Perkin Elmer gas chromatograph calibrated towards a gas mixture of known composition. The Gas Hourly Space Velocity (GHSV) is between 3,600 and 14,400 h⁻¹.

It was firstly checked if a Zn/Al-based catalyst, according to EP 2,141,118, shows a significant activity in MTS operating conditions. This Zn/Al calcined HT precursor has a molar ratio equal to 3 and is doped with 2 wt.% K, (reported in the text as "ZA3K").

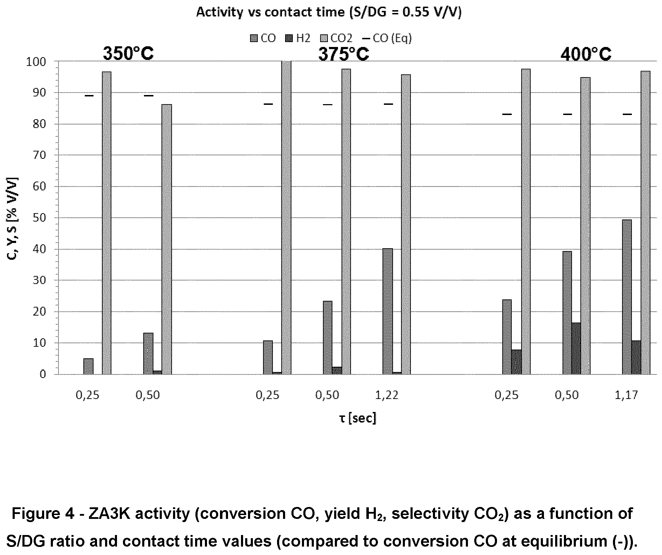

As shown on **Figure 4**, the 2 wt.% K-doped (ZA3K) sample is active at temperature higher than 350°C with a CO conversion that reaches 40 % in condition of larger excess of steam (S/DG = 0.55 v/v). Although the operating conditions are favourable, H₂ yield is always lower than the CO conversion value and reaches the highest value at 400°C, which may be explained by the presence of side reactions with H₂ consumption which are favoured by the high surface basic character, with formation of oxygenated product [K.J. Smith and R.B. Anderson, Can. J. Chem. Eng. 61 (1983) 40; Y.Okamoto et al., J. Phys. Chem. 87 (1983), 3740; C. E. Hofstadt et al., In Preparation of Catalysis III (G. Poncelet, P. Grange and P.A. Jacobs, Eds), Elsevier, Amsterdam (NL), 1983.]. This was confirmed by the HPLC analyses on the liquid condensates showing a significant presence of oxygenated products, mainly methanol.

Differently, Cu adding in the Zn/Al mixed oxide gives a real improvement in terms of CO conversion and H₂ yield. All results observed under all the operating conditions are summarized in the Table 2.

The samples obtained from carbonate-containing HT precursors (ZAC1c and ZAC2c) show the best performance in all the temperature range investigated, reaching the equilibrium values of CO conversion already at 300 °C. The ZAC2c sample has a Cu-content two times higher that ZAC1c (but remaining lower than 20 wt.%), trying to observe improved results at temperature between 250 and 300 °C.

**Table 2 - Summary of all results in terms of CO conversion and H₂ yield.**

| | | | | | | | | | | | | |
|---|---|---|---|---|---|---|---|---|---|---|---|---|
| | T (°C) | 250 | 250 | 250 | 300 | 300 | 300 | 350 | 350 | 350 | 250 | 300 |
| | S/DG (v/v) | 0.55 | 0.55 | 0.25 | 0.55 | 0.55 | 0.25 | 0.55 | 0.55 | 0.25 | 0.55 | 0.55 |
| | τ (s)* | 0.50 | 1.00 | 1.00 | 0.50 | 1.00 | 1.00 | 0.50 | 1.00 | 1.00 | 0.50 | 0.25 |
| ZAC1c | CO conv. (%) | 76.4 | 89.4 | 57.6 | 92.5 | 92.7 | 75.5 | 88.1 | 88.0 | 60.2 | 72.0 | 83.3 |
| | H₂ yield (%) | 75.6 | 88.7 | 54.7 | 90.6 | 91.7 | 72.6 | 85.9 | 86.1 | 5.8 | 70.1 | 82.8 |
| ZAC2c | CO conv. | 85.5 | 92.4 | 68.1 | 91.9 | 92.2 | 76.7 | 87.4 | 86.8 | 69.2 | 78.2 | 81.6 |
| | | 95.8 | 86.3 | 67.0 | 91.1 | 91.6 | 74.2 | 80.0 | 84.4 | 67.2 | 75.6 | 79.5 |
| ZAC3c | CO conv. | 24.1 | 37.3 | 20.7 | 47.5 | 61.8 | 43.0 | 68.8 | 74.5 | 56.9 | 17.4 | 26.8 |
| | | 24.0 | 37.3 | 23.9 | 44.9 | 63.5 | 41.6 | 69.2 | 74.7 | 57.4 | 15.8 | 24.6 |
| ZAC1cK | CO conv. | 32.4 | 49.7 | 30.9 | 61.9 | 76.3 | 58.7 | 79.8 | 85.5 | 65.2 | 22.0 | 33.8 |
| | | 33.5 | 50.1 | 29.4 | 62.3 | 75.8 | 59.6 | 79.6 | 86.5 | 66.2 | 21.8 | 35.7 |
| ZAC1s | CO conv. | 25.3 | 30.7 | 15.2 | 42.8 | 58.6 | 37.4 | 64.6 | 59.5 | 42.0 | 6.4 | 10.0 |
| | | 25.7 | 33.3 | 15.0 | 42.1 | 57.9 | 37.4 | 65.1 | 62.1 | 44.7 | 4.8 | 10.8 |
| ZAC2s | CO conv. | 15.5 | 15.4 | 8.2 | 47.7 | 36.2 | 27.4 | 64.3 | 55.3 | 39.4 | 10.1 | 10.9 |
| | | 15.4 | 15.2 | 8.0 | 46.1 | 34.9 | 22.9 | 66.2 | 54.9 | 39.1 | 11.9 | 11.2 |

| | | | | | | | | | | | | |
|---|---|---|---|---|---|---|---|---|---|---|---|---|
| * τ (s): Contact time (in seconds) | | | | | | | | | | | | |

As expected, the decrease of the S/DG ratio has a negative effect at all the temperatures, although it is significant only at 250 °C where the CO conversion decreases from 89 to 58 % (ZAC1c) and from 92 to 68 % (ZAC2c) by decreasing the S/DG value from 0.55 to 0.25.

At temperatures above 300 °C, the S/DG ratio effect on the activity of ZAC1c and ZAC2c catalysts is negligible, the CO conversion and the H₂ yield values being always close to the equilibrium values. The catalytic performances in the water gas shift reaction are strongly affected by the S/DG ratio for all the other catalysts.

The effect of the contact time (τ) is significant at 350°C for the carbonate-derived catalysts having a Cu content higher than 20 wt% and for all the silicate-derived catalysts.

Considering that the GHSV is higher than the typical industrial values, the activity of the carbonate-derived catalysts in the WGS reaction is slightly affected by the contact time: at 300 °C, because the CO conversion only decreased if the lowest contact time value (0.25 sec) is used, whereas at 250 °C it reaches 90 % (ZAC2c) with a contact time of 1.0 sec. At 300 °C, only a drastic decrease of contact time causes a decrease in both CO conversion and H₂ yield values. The selectivity is maximum in the syngas products, while lower by-products formation (i.e. methanol) and no carbon formation have been observed. The H₂ yield has practically the same trend of CO conversion: this result, together with the CO₂ selectivity values (always higher than 97 %), allows excluding the presence of significant side-reactions. Therefore, the activity for the tested samples seems to be affected by the thermodynamic (steam excess) and kinetics (contact time τ) parameters, in particular at 250°C. However, these samples are expected to reach the equilibrium value of CO conversion at 250 °C by using the conventional contact time values of industrial plants.

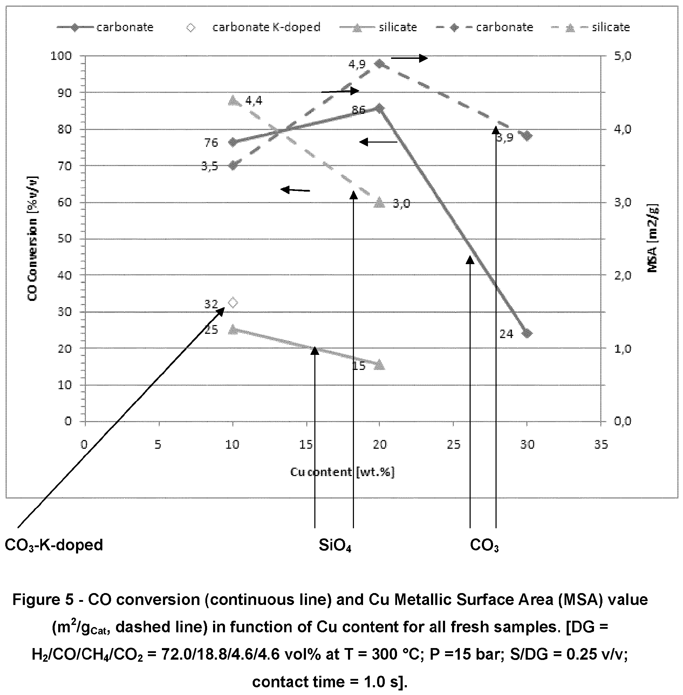

As shown on the graph of **Figure 5**, only a rough correlation exists between the Cu⁰-surface area and the catalytic activity (Fig.5).

For the samples obtained from carbonate-containing HT precursors, after a first slight increase, a dramatic decrease may be observed for the higher Cu-content, although the effect on the metallic surface area (MSA) is significantly lower than that on the catalytic activity.

For the samples obtained from silicate-containing HT-precursors the decrease of CO conversion is significantly higher in comparison to the former samples, than that scheduled on the basis of metallic surface area. This suggests the existence of synergetic effects between Cu- and Zn-containing phases, favoured in by the specific nature of the precursors.

### D] Catalyst Stability

The possible deactivation of the catalysts is studied at 300 °C for a period of 100 h, by using a most favourable contact time (1.0 sec), but in hard conditions in term of S/DG value (0.25 v/v). All the prepared catalysts show optimal stability with Time-on-Stream (tos), particularly the catalysts derived from the calcination of carbonate HT precursors. The same behaviour in terms of stability is observed for all the investigated samples, (Examples 1 to 6). As the equilibrium values are reached in more soft conditions, these results suggest a good performance of the catalysts in the middle temperature shift operating conditions.

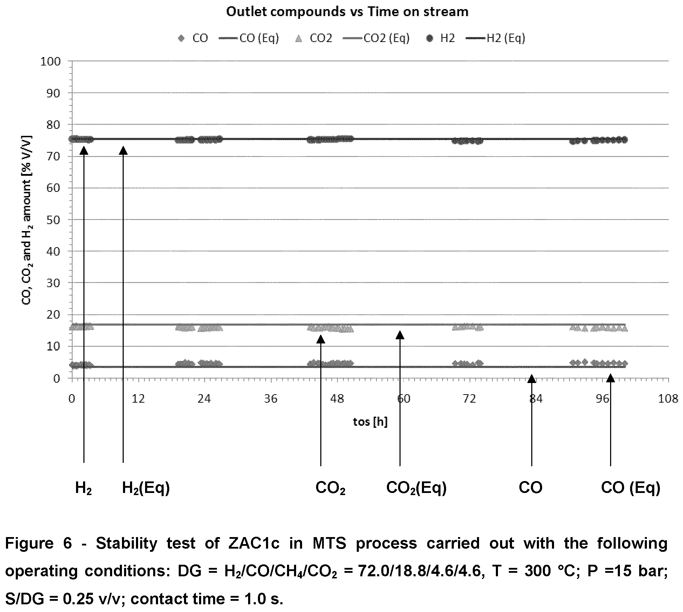

**Figure 6** shows the results using a ZAC1c sample. The long test demonstrates the stability of the performances of this catalyst with Time-on-stream (ToS) under pushed conditions in terms of the S/DG ratio (0.25v/v) and resident time (1.0 sec). The outlet DG composition is close to equilibrium one and remained stable during the long test. The CO amount increased only of 0.7 % after 100 h of ToS, evidencing a very stable behaviour of the ZAC1 c catalyst also under pushed shift conditions.

### E] Comparison between the catalysts according to the invention and with ZA3K catalyst

The activity and selectivity of the seven calcined HT catalysts in the WGS reaction carried out at medium temperature is studied. The following figures clearly show the best performance of Cu-containing catalysts derived from the calcination of carbonate-containing precursor, in terms of activity and selectivity, under typical industrial conditions, between 250°C and 350°C.

As shown in **Figure 4**, the sample without copper ZA3K shows a relatively good activity at high temperature, but the CO conversion is associated to a H₂ consumption, due to the significant presence of side-reactions with formation of oxygenated products (mainly methanol), attributable to its high basic character. Therefore, it seems not actually suitable as catalyst for the water gas shift process processes.

The catalysts are obtained from the hydrotalcite structure containing Cu, Zn, Al and having carbonate or silicate as interlayer anions, after calcination and metal activation by reduction.

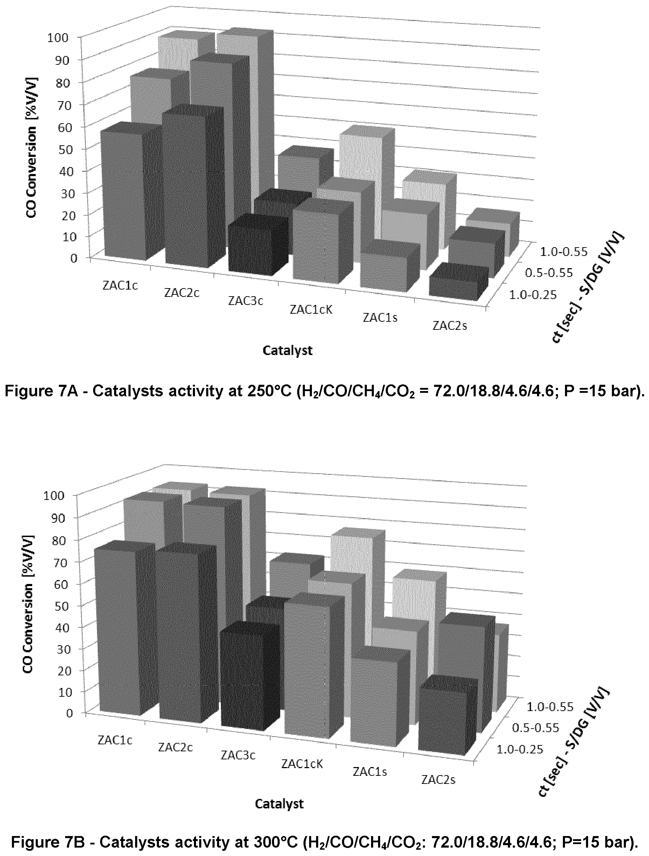

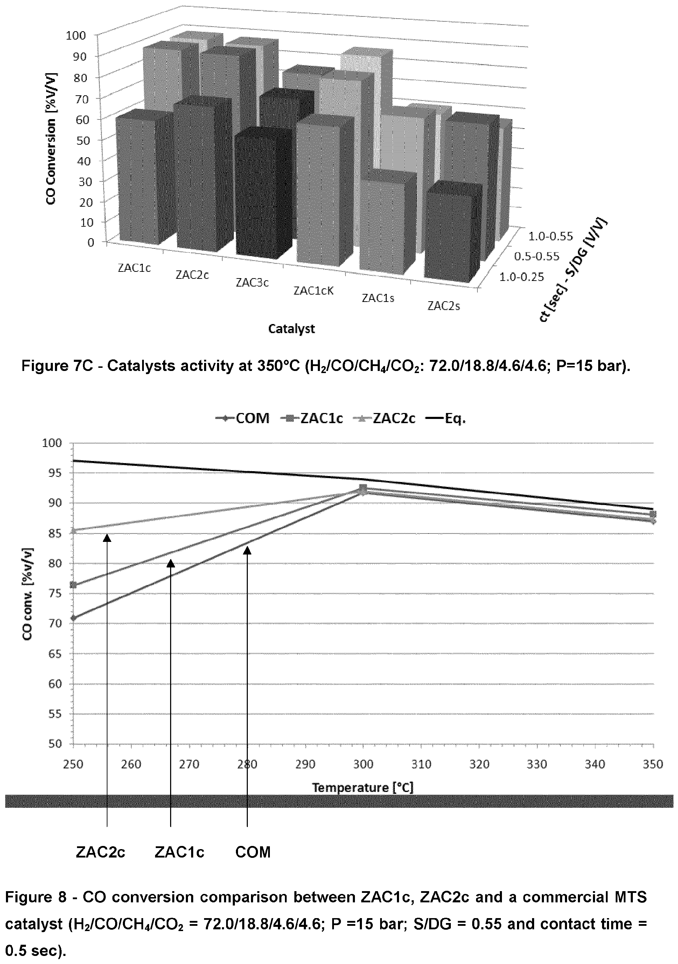

As shown in **Figures 7A, 7B** **and** **7C**, The ZAC catalysts obtained from silicate-containing HT precursors are selective but poorly active, while the ZAC catalysts obtained from carbonate-containing HT precursor with carbonate as interlayer anion, are highly active and selective. All the tested Cu-based catalysts obtained from carbonate-containig HT precursor show very good performances in all the operating conditions, regardless of S/DG ratio and contact time values adopted, particularly when the Cu content is lower than 20 wt.%. At 250 °C, a marked dependence on the operating conditions is observed, mainly related to the S/DG ratio. Both ZAC1c and ZAC2c are the most active catalysts, with values of CO conversion close to equilibrium ones, regardless of the S/DG ratio, when temperature is above 300 °C.

Moreover, both catalysts also show the same decrease of BET surface area after reaction, although practically without sintering of Cu⁰ crystallites, justifying their stable behaviour with time-on-stream after 100 h.

In conclusion, even if ZA3K catalyst is able to partially convert CO but with a significant H₂ consumption due to side-reactions, the various ZAC catalysts which all contains copper, show significantly better performances, due to the promoting effect of this metal. The ZAC2c sample may be considered the best catalyst, suitable also for non-conventional values of the S/DG ratio. The very good performances in the MTS conditions, associated with a good thermal stability, justify the use of ZAC catalysts in the WGS processes, in order to minimize sintering phenomena and side-reactions with H₂ consumption.

### F] Comparison with a commercial catalyst

As shown on **Figure 8**, the catalytic activities of catalysts prepared according to the present invention were compared with a widely diffuse commercial catalyst, evidencing better performances, approaching the equilibrium values, for the best catalysts, obtained from carbonate-containing HT precursors, with Cu-contents lower than 20 wt.%. These catalysts show also a very good stability with time-on-stream.

## Claims

1. Process for the production of a compound comprising the following steps:
- A step A during which an aqueous solution containing together, aluminium nitrate, zinc nitrate and copper nitrate is prepared by mixing said nitrate salts in the appropriate proportions, with water;
- A step B during which, said solution obtained at step A is mixed with an aqueous solution of hydrogenocarbonic acid sodium salt at 60°C, the pH being maintained between 8 and 10, preferably around 9, to produce a precipitate;
- A step C during which, said precipitate obtained at step B, is isolated by filtration, washed and then dried to form the expected hydrotalcite-type compound of formula (I):
[CuₓZn_{y}Al_{w}(OH)₂] ^{(2x+2y+3w-2)+} (A²⁻)_{(2x+2y+3w-2)/2}, k H₂O (I)
wherein:
- (A²⁻) represents either a carbonate anion or a silicate anion,
- x > 0,
- y >0,
- w >0,
- (x + y) = (1-w),
- 1 < [(x+y)/w] < 5, and
- 1/99 ≤ x/y ≤ 1/2.
- A step F during which the powder of an hydrotalcite-type compound of said formula (I) is calcined at 550°C for 6 hours;
- A step G during which the calcined powder obtained at step F is reduced with hydrogen, at a temperature of less than 230°C.

2. Process according to claim 1, wherein the atomic ratio x/y ≤ 1/5.

3. Process according to claim 1, wherein the atomic ratio x/y ≥1/10.

4. Process according to one of claims 1 to 3, wherein the atomic ratio [(x+y)/w] is higher or equal to 2.

5. Process according to one of claims 1 to 3, wherein the atomic ratio [(x+y)/w] is less or equal to 3.

6. Process according to claim 1, wherein the process comprises:
- A step D during which said hydrotalcite-type compound of said formula (I) as hereinabove defined, obtained at step C, is grinded to form a powder of particles of said hydrotalcite-type compound of said formula (I).

## Patentansprüche

1. Verfahren zur Herstellung einer Verbindung, umfassend die folgenden Schritte:
- einen Schritt A, während dessen eine wässrige Lösung, enthaltend zusammen Aluminiumnitrat, Zinknitrat und Kupfernitrat durch Mischen der Nitratsalze in geeigneten Proportionen mit Wasser zubereitet wird;
- einen Schritt B, während dessen die Lösung, erhalten in Schritt A, mit einer wässrigen Lösung aus Hydrogencabonsäure-Natriumsalz bei 60 °C gemischt wird, wobei der pH-Wert zwischen 8 und 10, vorzugsweise um 9 gehalten wird, um eine Ausfällung herzustellen;
- einen Schritt C, während dessen die Ausfällung, erhalten in Schritt B, durch Filtrierung isoliert, gewaschen und dann getrocknet wird, um die erwartete Verbindung vom Typ Hydrotalcit der Formel (I) zu bilden:
[CuₓZn_{y}Al_{w}(OH)₂]^{(2x+2y+3w-2)+} (A²⁻)_{(2x+2y+3w-2)/2}, k H₂O (I)
wobei:
- (A²⁻) entweder ein Carbonatanion oder ein Silikatanion darstellt,
- x > 0,
- y > 0,
- w > 0,
- (x + y) = (1 - w),
- 1 < [(x + y)/w] < 5, und
- 1/99 ≤ x/y ≤ 1/2.
- einen Schritt F, während dessen das Pulver einer Verbindung vom Typ Hydrotalcit der Formel (I) bei 550 °C während 6 Stunden kalziniert wird;
- einen Schritt G, während dessen das kalzinierte Pulver, erhalten in Schritt F, mit Wasserstoff bei einer Temperatur von weniger als 230 °C reduziert wird.

2. Verfahren nach Anspruch 1, wobei das Atomverhältnis x/y ≤ 1/5.

3. Verfahren nach Anspruch 1, wobei das Atomverhältnis x/y ≥ 1/10.

4. Verfahren nach einem der Ansprüche 1 bis 3, wobei das Atomverhältnis [(x+y)/w] größer als oder gleich wie 2 ist.

5. Verfahren nach einem der Ansprüche 1 bis 3, wobei das Atomverhältnis [(x+y)/w] kleiner als oder gleich wie 3 ist.

6. Verfahren nach Anspruch 1, wobei das Verfahren Folgendes umfasst:
- einen Schritt D, während dessen die Verbindung vom Typ Hydrotalcit der Formel (I), wie oben definiert, erhalten in Schritt C, gemahlen wird, um ein Pulver aus Partikeln der Verbindung vom Typ Hydrotalcit der Formel (I) zu bilden.

## Revendications

1. Procédé de production d'un composé comprenant les étapes suivantes :
- une étape A durant laquelle une solution aqueuse contenant ensemble, du nitrate d'aluminium, du nitrate de zinc et du nitrate de cuivre est préparée par mélange desdits sels de nitrate en les proportions appropriées, avec de l'eau ;
- une étape B durant laquelle, ladite solution obtenue à l'étape A est mélangée avec une solution aqueuse de sel sodique d'acide hydrogénocarbonique à 60 °C, le pH étant maintenu entre 8 et 10, de préférence autour de 9, pour produire un précipité ;
- une étape C durant laquelle, ledit précipité obtenu à l'étape B, est isolé par filtration, lavé puis séché pour former le composé de type hydrotalcite de formule (I) escompté :
[CuₓZn_{y}Al_{w}(OH)₂]^{(2x+2y+3w-2)+} (A²⁻)_{(2x+2y+3w-2)/2}, k H₂O (I)
dans lequel :
- (A²⁻) représente un anion carbonate ou un anion silicate,
- x > 0,
- y > 0,
- w > 0,
- (x + y) = (1 - w),
- < [(x + y)/w] < 5, et
- 1/99 ≤ x/y ≤ 1/2.
- une étape F durant laquelle la poudre d'un composé de type hydrotalcite de ladite formule (I) est calcinée à 550 °C pendant 6 heures ;
- une étape G durant laquelle la poudre calcinée obtenue à l'étape F est réduite avec de l'hydrogène, à une température inférieure à 230 °C.

2. Procédé selon la revendication 1, dans lequel le rapport atomique x/y ≤ 1/5.

3. Procédé selon la revendication 1, dans lequel le rapport atomique x/y ≥ 1/10

4. Procédé selon l'une quelconque des revendications 1 à 3, dans lequel le rapport atomique [(x + y)/w] est supérieur ou égal à 2.

5. Procédé selon l'une quelconque des revendications 1 à 3, dans lequel le rapport atomique [(x + y)/w] est inférieur ou égal à 3.

6. Procédé selon la revendication 1, dans lequel le procédé comprend :
- une étape D durant laquelle ledit composé de type hydrotalcite de ladite formule (I) tel que défini ci-dessus, obtenu à l'étape C, est broyé pour former une poudre de particules dudit composé de type hydrotalcite de ladite formule (I).
